# DEMANDE DE BREVET EUROPEEN

(11) **EP 0 953 332 A1**
(43) Date de publication de la demande: **03.11.1999**
(21) Numéro de dépôt: 99400892.8
(22) Date de dépôt: 12.04.1999
(51) Int. Cl.: A61K 7/032, A61K 7/06

(54) **Maquillage perlé des fibres kératiniques**

(30) Priorité: 15.04.1998 FR 9804687
(71) Demandeur: L'OREAL, 75008 Paris (FR)
(72) Inventeur: Auguste, Frédéric, 94550 Chevilly-Larue (FR); Bara, Isabelle, 75013 Paris (FR)
(74) Mandataire: Lhoste, Catherine

(57) **Abrégé**

L'invention a pour objet un procédé de maquillage des fibres kératiniques sensiblement longitudinales ou équivalents, comprenant l'application d'une composition comprenant au moins une dispersion aqueuse de particules de polymère filmogène, la composition présentant une viscosité, mesurée à un cisaillement de 500 s⁻¹, allant de 0,001 Pa.s à 2 Pa.s, la composition étant appliquée sur lesdites fibres kératiniques à une vitesse selon laquelle le film déposé sur les dites fibres kératiniques présente des perles réparties le long de ladite fibre kératinique.

## Description

La présente invention a pour objet une composition pour le maquillage des fibres kératiniques, notamment des cils et des cheveux, contenant une dispersion aqueuse de polymère, l'utilisation d'une telle composition pour le maquillage des fibres kératiniques, ainsi un procédé de maquillage de ces dernières. La composition et le procédé de maquillage selon l'invention sont plus particulièrement destinés aux fibres kératiniques sensiblement longitudinales tels que les cils, les sourcils et les cheveux, ainsi qu'aux postiches (perruques) et aux faux-cils. Plus spécialement, l'invention porte sur un mascara.

Les mascaras sont couramments préparés selon deux types de formulations: les mascaras aqueux, dits mascaras crèmes, sont sous forme d'émulsion de cires dans l'eau; les mascaras anhydres ou à faible teneur en eau, dits mascaras waterproofs, sont sous forme de dispersions de cires dans des huiles.

Les mascaras crèmes conventionnels se répartissent en général bien le long du cil en formant un dépôt homogène sur le cil et produisent un effet d'allongement du cil satisfaisant. Cependant, ces mascaras présentent l'inconvénient d'être peu résistants à l'eau et à l'humidité ambiante (pluie, larmes, bains) et présentent de mauvaises propriétés mécaniques comme la résistance aux frottements, notamment lors du passage des mains sur les paupières.

Les mascaras waterproofs conventionnels ont une bonne résistance à l'eau et à l'humidité ambiante mais présentent de mauvaises propriétés quant au dépôt et à la répartition du produit le long du cil. Ils nécessitent en outre l'utilisation de démaquillants spécifiques à base d'huile en raison de leur forte rémanence à l'eau.

ll est d'usage courant de réaliser ces compositions de mascara avec au moins un polymère filmogène pour permettre notamment un gainage du cil. Ainsi, il a été décrit dans la demande de brevet japonais JP-A-57-62216 et dans le brevet US-A-4423031 des compositions de mascara aqueuses contenant comme polymère filmogène un polymère acrylique sous forme de particules en dispersion aqueuse. L'application de telles compositions sur les cils conduit à la formation de films continus, adhérents sur les cils et résistants à l'eau.

Le but recherché par la présente invention est de réaliser un nouveau type de maquillage des cils différent de ceux obtenus jusqu'à présent avec les mascaras courants.

La demanderesse a constaté qu'un nouveau type de maquillage des fibres kératiniques pouvait être obtenu en appliquant dans des conditions particulières, une composition comprenant une dispersion aqueuse de particules de polymères filmogène, ayant une rhéologie choisie. On a ainsi constaté qu'un procédé particulier de maquillage conduit à la formation d'un dépôt discontinu sur les fibres kératiniques sensiblement longitudinales, ce dépôt étant sous forme de goutelettes successives qui, après séchage, forment une succession de perles réparties le long de la fibre kératinique. On obtient alors un maquillage ayant des perles réparties régulièrement sur la fibre kératinique.

Aussi, la présente invention a pour objet un procédé de maquillage des fibres kératiniques sensiblement longitudinales, comprenant l'application d'une composition comprenant au moins une dispersion aqueuse de particules de polymère filmogène, caractérisé par le fait que ladite composition présente une viscosité, mesurée à un cisaillement de 500 s⁻¹, allant de 0,001 Pa.s à 2 Pa.s, ladite composition étant appliquée sur lesdites fibres kératiniques à une vitesse selon laquelle le film déposé sur les dites fibres kératiniques présente des perles (ou petites boules) réparties le long de ladite fibre kératinique.

L'invention a également pour objet l'utilisation d'une composition telle que définie précédemment pour le maquillage des fibres kératiniques sensiblement longitudinales, le maquillage se présentant sous la forme d'un dépôt discontinu comportant des perles réparties le long de ladite fibre kératinique.

L'invention a encore pour objet une composition de maquillage comprenant au moins une dispersion aqueuse de particules de polymère filmogène et au moins un agent épaississant, caractérisé par le fait que ladite composition présente une viscosité, mesurée à un cisaillement de 500 s⁻¹, allant de 0,001 Pa.s à 2 Pa.s.

L'invention a aussi pour objet un mascara consistant en une composition telle que définie précédemment.

L'invention a encore pour objet une fibre maquillée comportant un film de polymère filmogène présentant des perles réparties le long de ladite fibre. La fibre peut être un faux-cil ou une fibre de postiche, notamment une fibre de perruque en matière synthétique.

Avantageusement, la composition selon l'invention a une viscosité, mesurée à un cisaillement de 500 s⁻¹, allant de 0,001 Pa.s à 1 Pa.s, et mieux allant de 0,01 Pa.s à 0,5 Pa.s. La viscosité est mesurée à 20 °C avec un viscosimètre rotatif à gradient de vitesse RHEOMAT 115 équipé de cylindres coaxiaux.

Selon le procédé selon l'invention, la composition peut être déposée de préférence à une vitesse allant de 0,08 m.s⁻¹ à 1,2 m.s⁻¹ pour permettre le dépôt de la composition sous forme de goutelettes qui conduisent, après séchage, à la formation de perles sur la fibre kératinique. Ces perles peuvent être notamment de forme sensiblement sphérique, ovoïdale ou ellipsoïdale ; elles peuvent avoir un diamètre allant environ de 0,1 mm à 5 mm, et de préférence de 0,1 à 2 mm.

La composition utilisée dans le procédé selon l'invention contient un polymère filmogène se présentant sous la forme de particules en dispersion aqueuse pour permettre le dépôt d'un film sur la fibre kératinique.

Parmi les polymères filmogènes utilisables dans le cadre de la présente invention, on peut citer les polymères synthétiques, de type radicalaire ou de type polycondensat, les polymères d'origine naturelle, et leurs mélanges.

Par polymère radicalaire, on entend un polymère obtenu par polymérisation de monomères à insaturation notamment éthylénique, chaque monomère étant susceptible de s'homopolymériser (à l'inverse des polycondensats).

Les polymères de type radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

On utilise de préférence des polymères radicalaires anioniques, c'est-à-dire des polymères formés à partir de monomères ayant au moins un groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1 -C20, de préférence en C1-C8, des (méth)acrylates d'aryle, en particulier d'aryle en C6-C10, des (méth)acrylates d'hydroxyalkyle, en particulier d'hydroxyalkyle en C2-C6 .
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodéca-noate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère acrylique utilisable selon l'invention, on peut citer ceux vendus sous les dénominations NEOCRYL XK-90®, NEOCRYL A-1070®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.
Le polyuréthanne peut être, par exemple, un copolymère polyuréthanne, poly-urée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsi-loxane ou polyméthylphénylsiloxane, et/ou
- au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.
Comme polyuréthanne utilisable selon l'invention on peut citer ceux commercialisés sous les dénominations NEOREZ R-981^{®}, NEOREZ R-974^{®} par la société ZENECA, les SANCURE 815^{®}, SANCURE 875^{®}, SANCURE 2060^{®}, SANCURE 2255^{®}, SANCURE 861^{®} par la société SANNCOR.

Parmi les polycondensats, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides: l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi: l'éthylène glycol, le diéthylène glycol, le triéthylène glycol, le 1,3-propanediol, le cyclohexane diméthanol, le 1,4-butanediol.
Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la mo-noéthanolamine.

Le polyester peut en outre comprendre au moins un monomère portant au moins un groupement -SO₃M, avec M représentant un atome d'hydrogène, un ion ammonium NH₄+ ou un ion métallique, comme par exemple un ion Na⁺, Li⁺, K⁺, Mg²⁺, Ca²⁺, Cu²⁺, Fe²⁺, Fe³⁺. On peut utiliser notamment un monomère aromatique bifonctionnel comportant un tel groupement-SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO3M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et est de préférence de 20 à 150 nm.

Le polymère en dispersion aqueuse peut être présent dans la composition selon l'invention en une teneur allant de 1 % à 60 % en poids, de préférence de 5 % à 40% en poids de matière sèche de polymères filmogènes par rapport au poids total de la composition.

Le milieu de la composition de l'invention est avantageusement un milieu aqueux contenant de l'eau.

Pour conférer à la composition utilisée dans le procédé selon l'invention la viscosité requise pour obtenir le dépôt sous forme de goutelettes, la composition peut contenir un agent épaississant permettant d'ajuster la viscosité voulue.

Parmi les agents épaississants utilisables selon l'invention, on peut citer :
- les épaississants cellulosiques hydrosoluble tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁ -C₆. On peut mentionner à titre d'exemple, les groupements hydroxymé-thyle, hydroxyéthyle, hydroxypropyle et hydroxybutyle. De telles gommes de guar sont notamment vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.
- les gommes de xanthane, de caroube, de scléroglucane, de gellane, de rham-san, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goo-drich,
- les polymères poly(méth)acrylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou de "Salcare SC95" par la Société Allied Colloïd, ou encore
- les homopolymères réticulés de chlorure de méthacryloyloxyéthyltriméthylam-monium vendus sous la dénomination de "Salcare SC95" par la Société Allied Colloïd.
- les polyuréthanes associatifs tel que celui vendu sous la dénomination "SER AD FX 1100" par la société SERVO.

Selon l'invention, l'agent épaississant est de préférence choisi parmi la gomme de xanthane, la laponite, les polyuréthanes associatifs, les épaississants cellulosiques, notamment l'hydroxyéthyl cellulose.

Dans la composition utilisée selon l'invention, l'agent épaississant peut être présent en une teneur telle que la composition présente la viscosité requise telle que définie précédemment. La teneur en agent épaississant peut par exemple aller de 0,01 % à 10 % en poids, par rapport au poids total de la composition, et de préférence de 0,1 % à 5 % en poids.

Avantageusement, la composition selon l'invention peut comprendre les associations polymères filmogènes / agents épaississants suivantes:
- lorsque le polymère filmogène est un polyester, et en particulier un sulfopolyes-ter, on utilise de préférence un agent épaississant choisi parmi les dérivés de cellulose, et notamment l'hydroxyéthyl cellulose, et la gomme de xanthane;
- lorsque le polymère filmogène est un polyuréthane, et en particulier un polyester-polyuréthane, on utilise de préférence un agent épaississant choisi parmi les dérivés de cellulose, et notamment l'hydroxyéthyl cellulose, les polyuréhanes associatifs, les argiles, notamment la laponite;
- lorsque le polymère filmogène est un polymère acrylique, et en particulier un acrylate, on utilise de préférence un agent épaississant choisi parmi les dérivés de cellulose, et notamment l'hydroxyéthyl cellulose, les argiles, notamment la laponite, la gomme de xanthane.

La composition peut en outre comprendre d'autres ingrédients habituellement utilisés en cosmétiques. De tels ingrédients peuvent être notamment des charges, des pigments, des cires, des tensio-actifs, des conservateurs, des huiles, des agents hydratants qui sont bien connus de l'état de la technique. Bien entendu, l'homme du métier veillera à choisir ce ou ces éventuels additifs et/ou leur quantité, de manière telle que les propriétés avantageuses du film déposé sur les fibres kératiniques soient conservées.

La composition convient parfaitement comme mascara pour les cils, les cheveux, les sourcils. Elle peut être appliquée sur les fibres kératiniques à l'aide de tout applicateur connu de l'homme du métier tels que les brosses à mascaras, les applicateurs à embout mousse, à plume floquée, en feutre, les pinceaux. L'applicateur doit être choisi de telle sorte que le dépôt de la composition selon le procédé de l'invention permette la formation de perles comme décrit précédemment.

On va maintenant donner des exemples illustrant la présente invention sans toutefois la limiter.

### Exemples 1 à 3 :

On a préparé des compositions filmogènes ayant la constitution suivante:
- dispersion aqueuse (P1) de polyester-polyuréthane à 35 % de matière active(MA) vendu sous la dénomination SANCURE 815 par la société SANNCOR 21 g MA
- agent épaississant (E) x g
- pigments 4 g
- propylène glycol 4 g
- eau qsp 100 g

On a utilisé les agents épaississants suivants :
- E1: hydroxyéthyl cellulose (Cellosize QP 4400H de Union Carbide)
- E2 : polyuréthane associatif (SER AD FX-1100 de SERVO)
- E3: laponite XLG de Laporte

| **Composition** | **épaississant en %** | **viscosité (en Pa.s.)** |
|---|---|---|
| **1a** | E1 - 0,2 | 0,032 |
| **1b** | E1 - 0,4 | 0,051 |
| **1c** | E1 - 0,6 | 0,074 |
| **1d** | E1 - 0,8 | 0,1 |
| **1e** | E1 - 1 | 0,12 |
| **2a** | E2 - 0,2 | 0,037 |
| **2b** | E2 - 0,4 | 0,21 |
| **2c** | E2 - 0,6 | 0,86 |
| **2d** | E2 - 0,8 | 1,62 |
| **3a** | E3 - 0,6 | 0,044 |
| **3b** | E3 - 0,8 | 0,074 |
| **3c** | E3 - 1 | 0,14 |

La teneur en agent épaississant est exprimée en pourcentage de poids par rapport au poids total de la composition.
La viscosité de chaque composition a été mesurée à 20 °C à l'aide d'un viscosimètre rotatif à gradient de vitesse RHEOMAT 115 équipé de cylindres coaxiaux

On a placé chaque composition dans un tube ouvert à chacune de ses extrémités. On a fait passer un cheveu à travers le tube rempli à la vitesse de 0,1 m/s. On a constaté que le cheveu maquillé avec chaque composition, à la sortie du tube, présentait des perles réparties régulièrement le long du cheveu.

### Exemples 4 à 6 :

On a préparé des compositions ayant la constitution suivante :
- dispersion aqueuse (P2) de copolymère acrylate à 45 % MA vendu sous la dénomination NEOCRYL A-1070 par la société ZENECA 27 g MA
- agent épaississant (E) x g
- pigments 4 g
- propylène glycol 4 g
- eau qsp 100 g

On a utilisé les agents épaississants suivants :
- E1 et E3 comme définis aux exemples 1 à 3
- E4 : gomme de xanthane de la société RHODIA

| **Composition** | **épaississant en %** | **viscosité (en Pa.s.)** |
|---|---|---|
| **4a** | E1 - 0,2 | 0,039 |
| **4b** | E1 - 0,4 | 0,12 |
| **4c** | E1 - 0,6 | 0,23 |
| **5a** | E3 - 0,2 | 0,023 |
| **5b** | E3 - 0,4 | 0,27 |
| **6a** | E4 - 0,6 | 0,092 |
| **6b** | E4 - 0,8 | 0,14 |
| **6c** | E4 - 1 | 0,19 |

En opérant dans les mêmes conditions indiquées aux exemples 1 à 3, on a constaté que le cheveu maquillé avec chaque composition, à la sortie du tube, présentait des perles réparties régulièrement le long du cheveu.

### Exemples 7 et 8 :

On a préparé des compositions filmogènes ayant la constitution suivante:
- sulfopolyester hydrodispersible vendu sous la dénomination AQ55S par la société EASTMAN KODAK 30 g
- agent épaississant x g
- pigments 4 g
- propylène glycol 4 g
- eau qsp 100 g

| **Composition** | **épaississant en %** | **viscosité (en Pa.s.)** |
|---|---|---|
| **7a** | E4 - 0,8 | 0,072 |
| **7b** | E4 - 1 | 0,083 |
| **7c** | E4 - 1,2 | 0,1 |
| **7d** | E4 - 1,4 | 0,1 |
| **7e** | E4 - 1,8 | 0,11 |
| **8a** | E1 - 1 | 0,16 |
| **8b** | E1 - 1,2 | 0,23 |
| **8c** | E1 - 1,4 | 0,26 |
| **8d** | E1 - 1,6 | 0,28 |
| **8e** | E1 - 1,8 | 0,315 |

En opérant dans les mêmes conditions indiquées aux exemples 1 à 3, on a constaté que le cheveu maquillé avec chaque composition, à la sortie du tube, présentait des perles réparties régulièrement le long du cheveu.

## Revendications

1. Procédé de maquillage des fibres kératiniques sensiblement longitudinales ou équivalents, comprenant l'application d'une composition comprenant au moins une dispersion aqueuse de particules de polymère filmogène, caractérisé par le fait que ladite composition présente une viscosité, mesurée à un cisaillement de 500 s⁻¹, allant de 0,001 Pa.s à 2 Pa.s, ladite composition étant appliquée sur lesdites fibres kératiniques à une vitesse selon laquelle le film déposé sur les dites fibres kératiniques présente des perles réparties le long de ladite fibre kératinique.

2. Procédé selon la revendication 1, caractérisé par le fait que la composition a une viscosité allant de 0,001 Pa.s à 1 Pa.s.

3. Procédé selon la revendication 1, caractérisé par le fait que la composition a une viscosité allant de 0,01 Pa.s à 0,5 Pa.s.

4. Procédé selon l'une quelconque des revendications précédentes, caractérisé par le fait que ladite composition est déposée à une vitesse allant de 0,08 m.s⁻¹ à 1,2 m.s⁻¹.

5. Procédé selon l'une quelconque des revendications précédentes, caractérisé par le fait que le polymère filmogène est choisi dans le groupe formé par les polymères radicalaires, les polycondensats, les polymères d'origine naturelle et leurs mélanges.

6. Procédé selon la revendication 5, caractérisé par le fait que les polymères radicalaires sont choisis dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

7. Procédé selon la revendication 6, caractérisé par le fait que les esters des acides carboxyliques insaturés α,β-éthyléniques sont choisis parmi les (méth)acrylates.

8. Procédé selon la revendication 5, caractérisé par le fait que les polycondensats sont choisis dans le groupe formé par les polyuréthanes, les polyesters, les po-lesters amides, les polyamides, les résines époxyesters.

9. Procédé selon la revendication 5, caractérisé par le fait que les polymères d'origine naturelle sont choisis dans le groupe formé par la résine shellac, la gomme de sandaraque, les dammars, les élémis, les copals, les polymères cellulosiques insolubles dans l'eau, et leurs mélanges.

10. Procédé selon l'une quelconque des revendications précédentes, caractérisé par le fait que la composition comprend, en outre, au moins un agent épaississant.

11. Procédé selon la revendication 10, caractérisé par le fait que l'agent épaississant est choisi dans le groupe formé par les épaississants cellulosiques, les gommes de guar, les gommes de xanthane, de caroube, de scléroglucane, de gellane, de rhamsan, de karoya, les alginates, la maltodextrine, l'amidon et ses dérivés, l'acide hyaluronique et ses sels, les argiles, les acides polyacryliques réticulés, les poly(méth)acrylates de glycéryle, la polyvinylpyrrolidone, l'alcool polyvinylique, les polymères et les copolymères réticulés d'acrylamide, les homopolymères réticulés de chlorure de méthacryloyloxyéthyltriméthylammonium, les polyuréthanes associatifs

12. Procédé selon la revendication 10 ou 11, caractérisé par le fait que l'agent épaississant est choisi dans le groupe formé par la gomme de xanthane, la lapo-nite, les polyuréthanes associatifs, les épaississants cellulosiques.

13. Utilisation d'une composition telle que définie selon l'une quelconque des revendications précédentes, pour le maquillage des fibres kératiniques sensiblement longitudinales, le maquillage se présentant sous la forme d'un dépôt discontinu comportant des perles réparties le long de ladite fibre kératinique.

14. Composition de maquillage comprenant au moins une dispersion aqueuse de particules de polymère filmogène et au moins un agent épaississant, caractérisé par le fait que ladite composition présente une viscosité, mesurée à un cisaillement de 500 s⁻¹, allant de 0,001 Pa.s à 2 Pa.s.

15. Composition selon la revendication 14, caractérisée par le fait que le polymère filmogène est défini selon l'une quelconque des revendications 5 à 9.

16. Composition selon les revendications 14 ou 15, caractérisée par le fait que l'agent épaississant est défini selon l'une quelconque des revendications 11 ou 12.

17. Mascara caractérisé par le fait qu'il consiste en une composition telle que définie selon l'une quelconque des revendications 14 à 16.

18. Fibre maquillée comportant un film de polymère filmogène présentant des perles réparties le long de ladite fibre.

19. Fibre maquillée selon la revendication 17, caractérisée par le fait que la fibre est un faux-cil ou une fibre de postiche.
